# EUROPEAN PATENT APPLICATION

(11) **EP 3 943 796 A1**
(43) Date of publication of application: **26.01.2022**
(21) Application number: 20847611.9
(22) Date of filing: 30.03.2020
(51) Int. Cl.: F16M 11/32, F16M 7/00, G01N 33/86

(54) **QUICK AND CONVENIENT THROMBOELASTOGRAPH LEVELING APPARATUS AND LEVELING METHOD**

(30) Priority: 29.07.2019 CN 201910686623
(71) Applicant: Hemoassay Science and Technology (Suzhou) Co., Ltd., Suzhou, Jiangsu 215000 (CN)
(72) Inventor: JIANG, Feng, Suzhou, Jiangsu 215000 (CN); CHEN, Jienian, Suzhou, Jiangsu 215000 (CN)
(74) Representative: Wang, Bo
(86) International application number: PCT/CN2020/082089
(87) International publication number: WO 2021/017506

(57) **Abstract**

The invention discloses a leveling device and a leveling method of a portable thromboelastometer which comprises a bottom plate and at least three bottom plate-leveling mechanisms arranged at three top corners of the bottom plate, wherein each bottom plate-leveling mechanism includes a rotationally-driving mechanism, a rotating shaft and a foot pad, the rotationally-driving mechanism is connected to the rotating shaft and used for driving the rotating shaft to rotate, the foot pad is connected with the rotating shaft and the bottom plate, the rotating shaft drives the foot pad to move up and down, and to adjust the levelness of the bottom plate. The invention achieves automatically adjusting the levelness of the bottom plate to ensure the adjustment accuracy and improve the adjustment efficiency.

## Description

### TECHNICAL FIELD OF THE INVENTION

The invention relates to a thromboelastometer, in particular to a leveling device and a leveling method of a portable thromboelastometer.

### DESCRIPTION OF THE PRIOR ART

A thromboelastometer is a medical device used to test whether blood can coagulate normally. It is generally necessary to detect the patient's blood coagulation data through the thrombo elastometer before performing an operation, and judge whether the patient's blood coagulation process is normal based on the detected blood coagulation data. Only when the blood is able to coagulate normally, the patient can be operated on. If the blood coagulation process is abnormal, the patient's blood will not be able to coagulate normally during the operation, leading to difficulty in hemostasis, which may endanger the patient's life.

As for the current thromboelastometer, before each blood test, it is necessary to manually calibrate the levelness of an elastometer base to keep the elastometer in a horizontal state. Since manual adjustment takes a long time and its accuracy is poor, it is necessary to develop a new leveling device to achieve automatically and accurately adjusting the levelness of the elastometer base.

### SUMMARY OF THE INVENTION

A objective of the invention is to overcome the defects of the prior art and provide a leveling device and a leveling method of a portable thromboelastometer.

To achieve the above object, the present invention provides the following technical solutions: a leveling device of a portable thromboelastometer, the device comprises a bottom plate and at least three bottom plate-leveling mechanisms arranged at three top corners of the bottom plate, wherein each bottom plate-leveling mechanism includes a rotationally-driving mechanism, a rotating shaft and a foot pad, the rotationally-driving mechanism is connected to the rotating shaft and used for driving the rotating shaft to rotate, the foot pad is connected with the rotating shaft and the bottom plate, the rotating shaft drives the foot pad to move up and down, and to adjust the levelness of the bottom plate.

Preferably, the rotationally-driving mechanism includes a driving source and a turn assembly, the turn assembly is connected to the driving source and the rotating shaft.

Preferably, the turn assembly includes a first pinion and a second pinion perpendicular to each other, one end of the first pinion is fixedly connected with the driving source, and the other end meshes with the second pinion, and the second pinion is also fixedly connected with the rotating shaft.

Preferably, the rotating shaft is threadedly connected with the foot pad, the rotating shaft axially rotates so as to drive the foot pad move up and down.

Preferably, the device further includes an anti-rotation post preventing the foot pad from axially rotating, the anti-rotation post is fixed on the bottom plate and fixedly connected with the foot pad.

Preferably, a rubber base covers the bottom of the foot pad, the bottom edge of the rubber base is a convex curved surface.

Preferably, a limit assembly used to prevent the rotating shaft from moving up and down is provided on the rotating shaft.

Preferably, the limit assembly includes a circlip and a bearing both fixedly encircling the rotating shaft, the circlip and the bearing are located on the upper and lower end surfaces of the bottom plate, respectively, and both arranged clinging to the the bottom plate.

Preferably, the device further includes a limit sensor used to limit the rotation amplitude of the rotating shaft, the limit sensor is connected with the rotationally-driving mechanism.

This present invention also discloses another technical solution: a leveling method of a portable thromboelastometer, comprising the rotationally-driving mechanism driving the rotating shaft to rotate, and the rotating shaft driving the foot pad to move up and down while rotating, the foot pad moving up and down while adjusting the levelness of the bottom plate.

The beneficial effect of the invention is that three leveling mechanisms are used for automatically adjusting the levelness of the bottom plate to ensure the adjustment accuracy and improve the adjustment efficiency.

### BRIEF DESCRIPTION OF THE DRAWINGS

FIG.1 is a schematic diagram of the leveling device according to the invention.
FIG.2 is a schematic diagram of one of the bottom plate-leveling mechanisms according to the invention.

Wherein:
10 - bottom plate; 20- bottom plate-leveling mechanisms; 21- rotating shaft; 22- foot pad; 23-driving source/motor; 24- motor housing; 25- first pinion; 26- second pinion; 27- circlip; 28-bearing; 30- anti-rotation post; 40- rubber base; 50- limit sensor.

### DETAILED DESCRIPTION OF THE INVENTION

The technical solutions of the embodiments in the invention will be clearly and completely described below in combination with the figures in the invention.

The leveling device and leveling method of a portable thromboelastometer disclosed by the invention is used for automatically and accurately adjusting the levelness of the thromboelastometer.

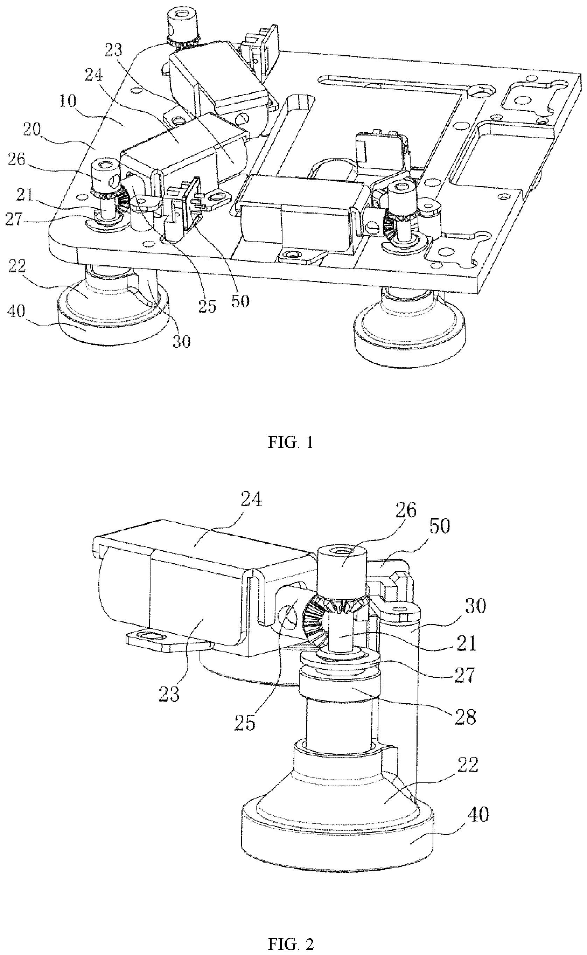

As shown in FIG. 1, the leveling device of a portable thromboelastometer disclosed by the invention includes a bottom plate 10 and at least three bottom plate-leveling mechanisms 20, wherein the bottom plate 10 is namely the bottom plate 10 of the portable thromboelastometer, certainly, the bottom plate 10 is not generally limited to a rectangular plate with four top corners. The three bottom plate-leveling mechanisms 20 are arranged at the three top corners of the bottom plate 10, respectively, so as to cooperatively adjust the levelness of the bottom plate 10, that is, to adjust the levelness of the portable thromboelastometer. Of course, as an alternative, all the four top corners of the bottom plate 10 can also be provided with a bottom plate-leveling mechanism 20, that is, the number of the bottom plate-leveling mechanism 20 is not limited to three.

The three bottom plate-leveling mechanisms 20 have the same structure, as shown in FIG. 2, each bottom plate-leveling mechanism 20 includes a rotationally-driving mechanism, a rotating shaft 21 and a foot pad 22, wherein the rotationally-driving mechanism is connected to the rotating shaft 21 and used for driving the rotating shaft 21 to rotate. In this embodiment, the rotationally-driving mechanism specifically includes a driving source 23 and a turn assembly, the driving source 23 is fixed on the bottom plate 10, when implemented, it can be a motor, and has a motor housing 24 covered outside the motor. The turn assembly is connected to the driving source 23 and the rotating shaft 21, and is used to convert the rotation direction of the motor shaft (not shown in the figures) of the motor, in this embodiment, the motor shaft is arranged horizontally, that is, the motor shaft rotates axially in the horizontal direction.

In this embodiment, the turn assembly specifically includes a first pinion 25 and a second pinion 26 perpendicular to each other, wherein one end of the first pinion 25 encircles the motor shaft, and the end surface of the other end is gear-shaped, when the motor shaft rotates, the first pinion 25 synchronously axially rotates in the horizontal direction. Since the first pinion 25 is arranged horizontally, correspondingly, the second pinion 26 is arranged vertically, and encircles the rotating shaft, the lower end surface of the second pinion 26 is also gear-shaped and meshes with the gear surfaces of the first pinion 25. In this way, when the first pinion 25 rotates axially in the horizontal direction, the second pinion 26 rotates axially in the vertical direction to realize a turn. While the second pinion 26 is rotating, it drives the rotating shaft 21 to synchronously axially rotate in the vertical direction.

The rotating shaft 21 is entirely vertically arranged, passes through the bottom plate 10, and passes through the lower end of the bottom plate 10 to be movably connected to the foot pad 22. In this embodiment, the rotating shaft 21 is a threaded rod with an external thread, and threadedly connected with the foot pad 22, in this way, when the rotating shaft 21 rotates, the foot pad 22 will be driven to move up and down, so as to adjust the levelness of the corresponding top angle of the bottom plate 10.

Preferably, in this embodiment, the rotating shaft 21 can only axially rotate in the vertical direction, but cannot move up and down. In order to prevent the rotating shaft 21 from moving in the vertical direction, a limit assembly is preferably provided on the rotating shaft 21. In this embodiment, the limit assembly includes a circlip 27 and a bearing 28, wherein the circlip 27 encircles the rotating shaft 21, and is located on the upper end surface of the bottom plate 10, arranged clinging to the upper end surface of the bottom plate 10, and used to prevent the rotating shaft 21 from moving downward during the rotation. The bearing 28 encircles the rotating shaft 21 and is located on the lower end surface of the bottom plate 10, arranged clinging to the lower end surface of the bottom plate 10, and used to prevent the rotating shaft 21 from moving upward during the rotation, that is, the circlip 27 and the bearing 28 are used to prevent the rotating shaft 21 from moving downward and upward during the rotation, respectively.

More preferably, in order to prevent the foot pad 22 from axially rotating while moving up and down, an anti-rotation post 30 is added to the foot pad 22 in the embodiment according to invention, specifically, the anti-rotation post 30 vertically passes through the bottom plate 10, and its lower end is fixedly connected with the foot pad 22, and the foot pad 22 levels the bottom plate through the anti-rotation post 30 while moving up and down.

Furthermore, in order to make the entire thromboelastometer forced uniformly, a rubber base 40 covers the bottom of the foot pad 23 in the embodiment according to the invention, the bottom edge of the rubber base 40 is a convex curved surface, so that when leveling the instrument, the bottom of the foot pad 23 and the work surface (not shown in the figures) are always in point contact, so as to make the entire thromboelastometer forced uniformly.

In addition, the leveling device of the embodiment according to the invention further includes a limit sensor 50 and a level sensor (not shown in the figures) connected to the rotationally-driving mechanism, and the limit sensor 50 is used to limit the rotation amplitude of the rotating shaft. The level sensor is used to detect the levelness of the bottom plate, and the rotationally-driving mechanism executes horizontal adjustment to the bottom plate 10 according to the induction signal of the level sensor.

The working principle of the leveling device of a portable thromboelastometer disclosed by the invention is as follows. The motor 23 drives the first pinion 25 to axially rotate in the horizontal direction. the first pinion 25 drives the second pinion 26 to axially rotate in the vertical direction while rotating, the second pinion 26 further drives the rotating shaft 21 to axially rotate in the vertical direction, the rotating shaft 21 drives the foot pad 22 to move up and down while rotating, the foot pad 22 drives the bottom plate 10 to move up and down through the anti-rotation post 30, so as to achieve the leveling effect of the bottom plate 10 by adjusting the levelness of the three top corners of the bottom plate 10.

The technical content and technical features of the invention have been disclosed as above, but a person skilled in the art may still make various replacements and modifications based on the teachings and disclosures of the invention without departing from the essence of the invention. Therefore, the protection scope of the invention should not be limited to the content disclosed in the embodiments, but should include various replacements and modifications that do not deviate from the invention, and are covered by the claims of this patent application.

## Claims

1. A leveling device of a portable thromboelastometer, **characterized in that** said device comprises a bottom plate and at least three bottom plate-leveling mechanisms arranged at three top corners of said bottom plate, wherein each bottom plate-leveling mechanism includes a rotationally-driving mechanism, a rotating shaft and a foot pad, said rotationally-driving mechanism is connected to said rotating shaft and used for driving said rotating shaft to rotate, said foot pad is connected with said rotating shaft and said bottom plate, said rotating shaft drives said foot pad to move up and down, and to adjust the levelness of said bottom plate.

2. The leveling device of a portable thromboelastometer according to claim 1, **characterized in that** said rotationally-driving mechanism includes a driving source and a turn assembly, said turn assembly is connected to said driving source and said rotating shaft.

3. The leveling device of a portable thromboelastometer according to claim 2, **characterized in that** said turn assembly includes a first pinion and a second pinion perpendicular to each other, one end of said first pinion is fixedly connected with said driving source, and the other end meshes with said second pinion, and said second pinion is also fixedly connected with said rotating shaft.

4. The leveling device of a portable thromboelastometer according to claim 1, **characterized in that** said rotating shaft is threadedly connected with said foot pad, said rotating shaft axially rotates so as to drive said foot pad move up and down.

5. The leveling device of a portable thromboelastometer according to claim 1 or 4, **characterized in that** said device further includes an anti-rotation post preventing said foot pad from axially rotating, said anti-rotation post is fixed on said bottom plate and fixedly connected with said foot pad.

6. The leveling device of a portable thromboelastometer according to claim 1, **characterized in that** a rubber base covers the bottom of said foot pad, the bottom edge of said rubber base is a convex curved surface.

7. The leveling device of a portable thromboelastometer according to claim 1, **characterized in that** a limit assembly used to prevent said rotating shaft from moving up and down is provided on said rotating shaft.

8. The leveling device of a portable thromboelastometer according to claim 7, **characterized in that** said limit assembly includes a circlip and a bearing both fixedly encircling said rotating shaft, said circlip and said bearing are located on the upper and lower end surfaces of said bottom plate, respectively, and both arranged clinging to the said bottom plate.

9. The leveling device of a portable thromboelastometer according to claim 1, **characterized in that** said device further includes a limit sensor used to limit the rotation amplitude of said rotating shaft, said limit sensor is connected with said rotationally-driving mechanism.

10. A leveling method based on said leveling device of a portable thromboelastometer according to any one of claims 1-9, **characterized in that** said method comprises the rotationally-driving mechanism driving the rotating shaft to rotate, and the rotating shaft driving the foot pad to move up and down while rotating, said foot pad moving up and down while adjusting the levelness of the bottom plate.
